# FASCICULE DE BREVET EUROPEEN

(11) **EP 0 397 227 B1**
(45) Date de publication et mention de la délivrance du brevet: **03.11.1993**
(21) Numéro de dépôt: 90200971.1
(22) Date de dépôt: 19.04.1990
(51) Int. Cl.: A61K 47/48, A61K 9/127, C12N 11/10, C12N 11/18, A23K 1/165, A61K 7/28

(54) **Procédé de préparation d'un produit particulaire antimicrobien, produit antimicrobien obtenu et applications**
Verfahren zur Herstellung von antimikrobiellen Partikeln, antimikrobielle Mittel und Anwendungen
Method of preparation of an antimicrobial particulate, its product and applications

(30) Priorité: 12.05.1989 FR 8906668
(43) Date de publication de la demande: 14.11.1990
(73) Titulaire: BIO SERAE LABORATOIRES SA, 12400 Saint Affrique (FR)
(72) Inventeur: Samain, Daniel, F-31400 Toulouse (FR); Nguyen, Frédérique, F-17640 Vaux sur Mer (FR); Degre, Michel Francois, F-11170 Alzonne (FR)
(74) Mandataire: Barre, Philippe

(56) Documents cités:
- EP-A- 0 062 434
- EP-A- 0 344 040
- WO-A-87/07838
- CHEMICAL ABSTRACTS, vol. 85, no. 25, résumé no. 190934h, Columbus, Ohio, US; L. BJORCK et al.: "An immobilized two-enzyme system for the activation of the lactoperoxidase antibacterial system in milk"

## Description

L'invention concerne un procédé de préparation d'un produit particulaire antimicrobien à partir du système antimicrobien constitué par l'enzyme lactopéroxydase, un donneur d'oxygène et le cas échéant un substrat oxydable. Elle vise le produit particulaire obtenu par mise en oeuvre du procédé et des applications de celui-ci dans le domaine pharmaceutique, de l'hygiène, de la désinfection et de la conservation.

Le système antimicrobien enzyme lactopéroxydase/donneur d'oxygène/substrat oxydable, dit système LP, est connu pour ses propriétés antimicrobiennes. Ce système est contenu dans les principales sécrétions des mammifères (lait, salive ou larmes) et assure leur protection ainsi que celle des muqueuses. Toutefois, ce système LP qui se présente sous la forme de molécules de petites tailles est très soluble et milieu aqueux de sorte que, en milieu humide ou au contact d'un corps hydraté, il n'est pas possible de limiter sa diffusion afin de concentrer son action sur une zone donnée. De plus, sa récupération doit faire appel à des techniques de séparation très sophistiquées.

La présente invention se propose de fournir à partir dudit système LP, un produit particulaire dont les composants protéiques soient insolubles en milieu aqueux, leur mobilité et leur diffusabilité pouvant être ajustées en fonction de l'application.

Un objectif essentiel de l'invention est d'obtenir le produit particulaire précité sans dénaturation du système LP, ni altération chimique de la ou des enzymes du système.

Un autre objectif est de permettre de donner au produit particulaire des propriétés complémentaires (charges électriques) adaptées aux applications envisagées.

Le procédé de préparation conforme à l'invention utilise le système LP précité dans lequel le donneur d'oxygène peut être, soit du péroxyde, soit des substances réactives dont l'une peut être une enzyme (glucose-oxydase, xantine-oxydase...), soit des micro-organismes (lactobacilles, streptocoques lactiques...). Ce système LP peut ou non comprendre un substrat oxydable selon l'application envisagée (thiocyanate, sel d'iode ou de brome...) : ce substrat n'est pas nécessaire dans le cas d'une application en milieu contenant déjà un tel substrat oxydable en quantité suffisante. Le procédé de l'invention se caractérise en ce que :
. l'on ancre au moins l'enzyme lactopéroxydase du système LP sur un vecteur particulaire dont chaque particule comprend un noyau constitué d'un polymère hydrophile polysaccharidique, une première couche lipidique comprenant des acides gras à longues chaînes liée chimiquement au noyau par des fonctions hydroxy ou fonctions dérivées et une seconde couche phospholipidique, de sorte que les molécules d'enzyme soient insérées dans la seconde couche phospholipidique et/ou dans la première couche lipidique,
. l'on conditionne en milieu non aqueux le vecteur particulaire précité et les molécules du système LP non intégrées dans ledit vecteur particulaire.

Dans le cas où le donneur d'oxygène contient comme subtance active une enzyme oxydase, celle-ci est de préférence également ancrée sur le vecteur particulaire.

Ainsi, le procédé de l'invention conduit à un produit particulaire antimicrobien dans lequel la ou les enzymes sont ancrées sur un vecteur particulaire insoluble en milieu aqueux, dont la taille et la densité peuvent être adjustées par une sélection appropriée du noyau polysaccharidique ; il est à noter que le vecteur est constitué d'éléments biocompatibles et ingérables (notamment métabolisables sans résidu) ce qui autorise une utilisation du produit dans les applications médicales et alimentaires.

On a pu constater expérimentalement que l'ancrage de l'enzyme dans les couches lipidiques et phospholipidiques est stable dans les conditions normales d'utilisation, le produit n'ayant aucune tendance à leur libération. Ces enzymes situées en surface du vecteur et non dénaturées conservent leur activité enzymatique pour catalyser la réaction d'oxydation du substrat par le donneur d'oxygène, ce qui conduit à l'action antimicrobienne connue du système LP. Cette activité est maintenue intégralement ou quasi-intégralement avec toutefois dans la plupart des cas une baisse cinétique due à la plus faible mobilité des composés.

Cependant, de façon inattendue, la cinétique in vitro ne semble pas affectée dans le cas où l'on greffe à la surface du noyau polysaccharidique des fonctions ioniques au moment où est constituée la première couche lipidique. Le caractère ionique sous-jacent ainsi conféré à cette première couche lipidique paraît avoir un effet favorable sur l'activité enzymatique de la lactopéroxydase qui compense la baisse de cinétique. Cette effet actuellement difficilement explicable a été mesuré par la méthode colorimétrique à l'orthophénylène diamine (constituant le substrat oxydable), le donneur étant pour ces mesures constitué par du péroxyde.

Le procédé de l'invention peut être utilisé pour préparer un système LP renforcé par de la lactoferrine LF, cette dernière étant également ancrée sur le vecteur particulaire. De même, des additifs peuvent être ajoutés au système et en particulier une enzyme superoxydismutase SOD ayant une fonction de protection du milieu, cette enzyme étant également ancrée sur le vecteur particulaire.

De préférence, chaque enzyme est ancrée indépendamment sur le vecteur particulaire de façon que chaque particule porte une seule enzyme, les produits particulaires ainsi préparés étant ensuite mélangés entre eux et avec les autres produits du système LP.

Dans certaines applications, il est intéressant de retarder la mise en activité des composés afin d'éviter un perte d'activité en dehors du site de l'application. Dans ce cas, selon un mode de mise en oeuvre possible du procédé, après ancrage, l'on enrobe les particules du vecteur particulaire au moyen d'une couche superficielle de polyoloside ou de substance colloïdale. En milieu non aqueux, cette couche superficielle isole les différentes substances réactives qui pourront être mélangées très longtemps avant utilisation du produit, l'activité étant amorcée lors de l'utilisation par hydratation et solubilisation de la couche superficielle de polyoloside. L'enrobage au moyen de cette couche peut être réalisé par des procédés de dragéification ou de "spray" classiques.

Par ailleurs, selon un autre mode de mise en oeuvre, l'on peut insérer des molécules amphiphiles électriquement chargées dans la seconde couche phospholipidique du vecteur particulaire, en vue de conférer une charge électrique aux particules. Il est ainsi possible de favoriser ou au contraire de défavoriser des phénomènes d'agrégation ou d'adhésion desdites particules, soit sur des surfaces, soit avec d'autres particules en suspension, soit encore avec des molécules solubilisées.

La méthode d'ancrage sur le vecteur particulaire de la ou des enzymes à ancrer peut être celle décrite dans la demande de brevet français n° 88.07110. Cet ancrage est en particulier réalisé :
. dans un premier temps, en greffant la couche lipidique autour du noyau polysaccharidique dans un milieu approtique non solvant des polysaccharidiques, pour former un noyeu acylé,
. ensuite, en mettant en présence dudit noyau acylé, d'une part les phospholipides de la seconde couche, d'autre part la ou les enzymes à ancrer, et en hydratant l'ensemble à une température voisine de la température de transition des phospholipides.

Dans le cas d'une couche lipidique à caractère ionique sous-jacent, le greffage de cette couche sur le noyau polysaccharidique consiste à faire réagir le noyau polysaccharidique avec un mélange d'un chlorure d'acide gras et d'un anhydride de diacide carboxylique en proportion minoritaire en particulier anhydride succinique.

En pratique, l'anhydride du diacide carboxylique est mélangé avec une teneur molaire inférieure à 40 % par rapport au chlorure d'acide gras (chlorure stéarique ou autre) en vue de préserver un caractère lipidique suffisant pour que la seconde couche phospholipidique puisse adhérer.

Le noyau polysaccharidique est dans chaque cas choisi en fonction de l'application. Il peut être naturel, son insolubilité étant due à sa nature chimique (cellulose, certaines variétés d'amidons telles que amylopectine) ou au contraire obtenue par réticulation synthétique (dextrane ou béta-amylose). La taille désirée est obtenue par broyage et tamisage appropriés. Dans le cas de noyaux réticulés, leur densité peut être ajustée en faisant varier le degré de réticulation lors de la polymérisation. La taille desdits noyaux polysaccharidiques peut varier entre 10⁻⁸m et 10⁻⁵m, les petites tailles étant intéressantes dans le cas des médicaments pour autoriser une certaine diffusion (par exemple pour traverser une barrière telle que la peau), les grosses tailles étant intéressantes pour empêcher la diffusion afin de concentrer l'activité et pour permettre le cas échéant une récupération des enzymes par voie physique (filtration, centrifugation ou décantation). En cas de noyaux humides, on réalise un séchage de ceux-ci avant fixation de la première couche, séchage qui peut par exemple être effectué par atomisation après polymérisation et fragmentation en vue d'éviter la formation d'agrégats.

La première couche lipidique est greffée en milieu non solvant (vis-à-vis des noyaux polysaccharidiques), notamment le dichlorométhane : les composés, constitués par les noyaux, un chlorure ou un anhydride d'acide (notamment stéarique) et une amine tertiaire jouant le rôle de catalyseurs (par exemple triéthylamine) sont dispersés dans ce milieu de façon à produire une réaction d'acylation à la périphérie de chaque noyau. L'épaisseur de cette couche peut être ajustée en adaptant la stoechiométrie et la durée de la réaction. De préférence, on adapte ces conditions pour obtenir une couche très mince, en particulier, monomoléculaire qui suffit à assurer l'ancrage de la seconde couche et de l'enzyme. De plus, la qualité de l'ancrage et le maintien de l'activité enzymatique paraissent favorisés par le greffage d'acides gras à longues chaînes (supérieures à 14 carbones). On greffe en pratique des acides gras à longueurs de chaînes comprises entre 14 et 22 pour éviter une rigidification de ladite couche au-delà de ce nombre. En particulier, des acides gras C18 (acide stéarique) donnent d'excellents résultats. Comme déjà indiqué, un caractère ionique sous-jacent peut être conféré à cette première couche en ajoutant de l'anhydride succinique en teneur minoritaire dans le milieu.

La seconde couche phospholipidique est de préférence réalisée à partir de phospholipides d'origine animale ou végétale telle que lecithine de soja, lecithine d'oeuf, lécithine du lait. Cette seconde couche peut être réalisée en mélangeant les noyaux acylés, les phospholipides et les enzymes à ancrer dans de l'eau maintenue à la température de transition des phospholipides et en agitant. La durée de formation de ladite couche est de l'ordre de 30 à 60 minutes. La stoechiomètrie est adaptée en fonction de la taille des noyaux acylés pour obtenir une monocouche de phospholipide (couche moléculaire). Cette couche possède un double rôle :
- permettre aux particules de se disperser de façon homogène en milieu aqueux (ce qui ne serait pas le cas en présence de la seule première couche rigoureusement hydrophobe ) ;
- améliorer l'ancrage de l'enzyme (ou autres éléments).

L'invention s'étend, en tant que produit nouveau, au produit particulaire antimicrobien obtenu par mise en oeuvre du procédé précité. Ce produit comprend une enzyme lactopéroxydase, un donneur d'oxygène et le cas échéant un substrat oxydable et est caractérisé en ce que, au moins l'enzyme lactopéroxydase du système est ancrée sur un vecteur particulaire dont chaque particule comprend un noyau constitué d'un polymère hydrophile polysaccharidique, une première couche lipidique liée chimiquement au noyau par des fonctions hydroxy ou fonctions dérivées et une seconde couche phospholipidique, les molécules d'enzyme étant insérées dans la seconde couche phospholipidique et/ou dans la première couche lipidique.

L'invention vise également des compositions pour l'application du produit précité, en particulier :
- composition médicamenteuse pour le traitement de la peau, des muqueuses ou du tube digestif,
- composition pour la décontamination microbienne de produits ou matériaux,
- dentifrice antiseptique.

Le produit particulaire conforme à l'invention associe les propriétés suivantes qui le rendent parfaitement adapté à ces applications :
- activité antimicrobienne efficace,
- biocompatibilité et caractère biomimétique (analogie de la seconde couche avec la membrane cellulaire lui permettant d'interagir avec cette dernière et de maintenir l'activité enzymatique à la surface même des tissus),
- diffusion limitée et excellente stabilité.

La description qui suit fournit des exemples de mise en oeuvre qui illustrent le procédé de l'invention et les performances des produits particulaires obtenus.

### Exemple 1

Cet exemple vise la préparation d'un produit particulaire antimicrobien de granulométrie relativement élevée (de 10⁻⁶ à 3.10⁻⁶m) en vue de présenter une faible diffusion.

### Préparation du noyau polysaccharidique

Dans un ballon de 1 litre, on mélange 150 g d'amidon type bétaamylose avec 150 ml d'une solution de soude 1 M. Lorsque la solution est homogène, on ajoute 10 ml d'épichlorydrine, on agite vigoureusement pendant 5 minutes, on chauffe à 80° C et on laisse polymériser à 80° C pendant 10 heures. On obtient un gel réticulé qui est lavé et remis en suspension dans 2 l d'eau, puis broyé mécaniquement (broyeur à hélice) et tamisé par centrifugation pour parvenir à une granulométrie comprise entre 10⁻⁶ et 3.10⁻⁶m.

On sèche le produit par atomisation et on obtient des particules sphéroîdes relativement souples en raison d'un degré de réticulation peu élevé.

### Greffage de la première couche lipidique

100 g des particules précédentes sont dispersées dans 1 litre de dichlorométhane. On ajoute dans le milieu 25 g de triethylamine et 60 g de chlorure d'acide stéarique. On mélange le milieu sous agitation lente à 37° C pendant 24 heures. On décante les particules et on les lave à l'éthanol.

Une analyse par la méthode de "Lauwerys" montre que l'acide stéarique (C18) est lié au noyau par des fonctions esters dérivées des fonctions hydroxy du noyau, avec une teneur pondérale de 2 % d'acide par rapport aux noyaux. Cette teneur est caractéristique d'une couche monomoléculaire. Les particules obtenues présentent un fort caractère hydrophobe que reflète le caractère total de l'acylation.

### Ancrage de la lactopéroxydase et formation de la seconde couche

De la lactopéroxydase est extraite d'un lactosérum bovin par chromatographie d'échange d'ions. Cette lactopéroxydase de pureté supérieure à 95 % est lyophilisée en vue d'être parfaitement sèche et stable.

Dans cet exemple, le système LP est constitué par ladite lactopéroxydase, par du thiocyanate de potassium comme substrat oxydable, et par le système glucose-oxydase comme donneur d'oxygène, seule la lactopéroxydase étant ancrée sur le vecteur particulaire.

Dans un ballon de 1 litre, on place 2 g des noyaux polysaccharidiques acylés précédemment préparés, 0,2 g de lyophilisat de lactopéroxydase et 0,2 g de lécithine hydrogénée, (origine animale : oeuf de poule, soumis à une hydrogénation). Le mélange est effectué à sec et on rajoute par petites fractions 400 ml d'eau distillée en agitant. La suspension est incubée à 37° C (température de transition de la lécithine) pendant 30 minutes.

Une partie de la suspension est centrifugée pour séparer les particules du milieu et l'activité enzymatique de la lactopéroxydase ainsi ancrée est analysée par la méthode à l'orthophénylène diamine. On constate que le pourcentage d'enzyme non ancrée sur le vecteur (restant dans la solution) est de 0,5 % (très faible perte) et que l'activité enzymatique est équivalente à 60 % de l'activité de la même quantité de lactopéroxydase à l'état libre. Ces résultats sont très satisfaisants et montrent que la lactopéroxydase n'est pas dénaturée, ni masquée par le vecteur (la baisse d'activité provenant, pour l'essentiel, de la moindre mobilité et dont d'un ralentissement de la cinétique).

L'autre partie de la suspension est lyophilisée et mélangée avec les autres constituants du système LP : thiocyanate de potassium (poudre sèche provenant du commerce) et système glucose-oxydase (provenant également du commerce et se présentant sous la forme de poudre), dans les proportions pondérales suivantes :
. 44,20 % de vecteur particulaire portant la lactopéroxydase,
. 11,06 % de thiocyanate de potassium,
. 44,20 % de glucose,
. 0,44 % d'oxydase.

Le produit granulaire ainsi préparé a été utilisé après hydratation pour tester son action antimicrobienne sur des bactéries du genre Listéria. Un dénombrement comparatif avec des cultures témoins a montré que l'activité antimicrobienne est très efficace (destruction totale des Listéria après 4 heures d'incubation à 37° C, alors que la population des témoins est conservée).

### Exemple 2

Les noyaux polysaccharidiques sont les mêmes que précédemment.

L'acylation est faite avec la même procédure mais avec une quantité moindre de chlorure d'acide stéarique 48 g et en ajoutant 4 g d'anhydride succénique afin d'apporter un caractère acide ionique sous-jacent à la première couche lipidique.

Le taux d'acide gras obtenu dans ce cas est de 1,6 % : la monocouche est composée par les acides gras avec certains sites occupés par des monoesters de l'acide succinique.

La seconde couche phospholipidique et l'ancrage de la lactopéroxydase sont réalisés comme à l'exemple 1.

Les analyses montrent dans ce cas que le pourcentage d'enzyme non ancrée sur le vecteur est légèrement inférieur (0,35 %) et surtout que l'activité enzymatique est équivalente à 100 % de celle de la même quantité d'enzyme à l'état libre. Ces essais semblent donc démontrer un effet d'activation de l'activité enzymatique de la lactopéroxydase par l'ionisation partielle de la première couche.

Le produit particulaire antimicrobien est préparé comme précédemment à partir des particules précitées et les autres composants du système LP.

### Exemple 3

La suspension du biovecteur chargé de lactopéroxydase fabriqué à l'exemple 1 est reprise avant lyophilisation. On y ajoute du mannitol en poids égal au double de poids de vecteur. On mélange sous agitation afin de dissoudre la mannitol et on atomise la suspension à basse température (35° C).

On obtient des particules dans lesquelles la seconde couche est entourée d'une pellicule d'enrobage de mannitol, qui masque l'effet enzymatique tant que cette pellicule très hydrosoluble n'est pas dissoute par hydratation.

Le produit particulaire antimicrobien est ensuite préparé avec les autres composants comme auparavant. Sa stabilité en milieu non aqueux est parfaite, aucune perte d'activité n'étant constatée sur de longues périodes de conservation.

## Revendications

1. Procédé de préparation d'un produit particulaire anti-microbien à partir du système anti-microbien enzyme lactoperoxydase/donneur d'oxygène/le cas échéant substrat oxydable, dit système LP, dans lequel le donneur d'oxygène est constitué soit par du peroxyde, soit par des substances réactives dont l'une peut être une enzyme, soit par des micro-organismes, ledit procédé étant destiné à modifier les propriétés physicochimiques du système LP en vue de le disposer sous forme de particules insolubles en milieu aqueux, ledit procédé étant caractérisé en ce que :
. l'on ancre au moins l'enzyme lactopéroxydase du système LP sur un vecteur particulaire dont chaque particule comprend un noyau constitué d'un polymère hydrophile polysaccharidique, une première couche lipidique comprenant des acides gras à longues chaînes, liée chimiquement au noyau par des fonctions hydroxy ou fonctions dérivées et une seconde couche phospholipidique, de sorte que les molécules d'enzyme soient insérées dans la seconde couche phospholipidique et/ou dans la première couche lipidique,
. l'on conditionne en milieu non aqueux le vecteur particulaire précité et les molécules du système LP non intégrées dans ledit vecteur particulaire.

2. Procédé de préparation selon la revendication 1, dans lequel le donneur d'oxygène contient comme substance active une enzyme oxydase, caractérisé en ce que l'on ancre également sur le vecteur particulaire lesdites molécules d'enzymes oxydases.

3. Procédé selon l'une des revendications 1 ou 2 pour la préparation d'un système LP renforcé par de la lactoferrine LF, caractérisé en ce que l'on ancre la LF sur le vecteur particulaire.

4. Procédé selon l'une des revendications 1, 2 ou 3 pour la préparation d'un système LP contenant comme protecteur du milieu une enzyme superoxydismutase SOD, caractérisé en ce que l'on ancre la SOD sur le vecteur particulaire.

5. Procédé de préparation selon l'une des revendications 1, 2, 3 ou 4, caractérisé en ce que l'on greffe à la surface du noyau polysaccharidique des fonctions ioniques en même temps que l'on constitue la première couche lipidique en vue de conférer un caractère ionique à cette couche lipidique.

6. Procédé de préparation selon l'une des revendications 1, 2, 3, 4 ou 5, caractérisé en ce que l'on insère des molécules amphiphiles électriquement chargées dans la seconde couche phospholipidique du vecteur particulaire, en vue de conférer une charge électrique aux particules.

7. Procédé de préparation selon l'une des revendications 1 à 6, dans lequel l'ancrage sur le vecteur particulaire, du ou des éléments à ancrer (enzyme(s) et le cas échéant LF, SOD) est réalisé :
. dans un premier temps en greffant la couche lipidique autour du noyau polysaccharidique dans un milieu aprotique non solvant des polysaccharidiques pour former un noyau acylé,
. ensuite, en mettant en présence dudit noyau acylé, d'une part les phospholipides de la seconde couche, d'autre part le ou les éléments à ancrer, et en hydratant l'ensemble à une température voisine de la température de transition des phospholipides.

8. Procédé de préparation selon les revendications 5 et 7 prises ensemble, caractérisé en ce que le greffage sur le noyau saccharidique de la couche lipidique et de ses fonctions ioniques consiste à faire réagir le noyau polysaccharidique avec un mélange d'un chlorure d'acide gras et d'un anhydride de diacide carboxylique en particulier anhydride succinique à teneur minoritaire.

9. Procédé de préparation selon l'une des revendications 1 à 8, caractérisé en ce que, après ancrage, l'on enrobe les particules du vecteur particulaire au moyen d'une couche superficielle de polyoloside ou de substance colloïdale.

10. Procédé de préparation selon l'une des revendications 1 à 9, dans lequel le noyau est réalisé à partir d'un polymère polysaccharidique du groupe suivant : amidon, dextrane, cellulose.

11. Procédé de préparation selon l'une des revendications 1 à 10, dans lequel la première couche lipidique est réalisée par réaction d'un chlorure ou d'un anhydride d'acide, catalysée par une amine tertiaire, ces composés étant dispersés dans le dichlorométhane en même temps que les noyaux saccharidiques.

12. Procédé de préparation selon la revendication 11, caractérisé en ce que l'on utilise pour réaliser la première couche un chlorure ou un anhydride d'acide gras à longue chaîne comprise entre 14 et 22 et notamment égale à 18.

13. Procédé de préparation selon l'une des revendications 1 à 12, dans lequel la seconde couche phospholipidique est réalisée à partir de phospholipides d'origine animale ou végétale telle que lécithine de soja, lécithine d'oeuf, lécithine du lait.

14. Produit particulaire antimicrobien comprenant une enzyme lactoperoxydase, un donneur d'oxygène contenant éventuellement une enzyme comme substance réactive, et le cas échéant un substrat oxydable, caractérisé en ce que au moins l'enzyme lactopéroxydase du système est ancrée sur un vecteur particulaire dont chaque particule comprend un noyau constitué d'un polymère hydrophile polysaccharidique, une première couche lipidique liée chimiquement au noyau par des fonctions hydroxy ou fonctions dérivées et une seconde couche phospholipidique, lesdites molécules d'enzyme étant insérées dans la seconde couche phospholipidique et/ou dans la première couche lipidique.

15. Produit particulaire antimicrobien selon la revendication 14, caractérisé en ce qu'il comprend, ancrées sur le vecteur particulaire, des molécules d'au moins un des composés suivants : lactoferrine LF, superoxydismutase SOD.

16. Produit particulaire antimicrobien selon l'une des revendications 14 ou 15, caractérisé en ce que la première couche lipidique présente un caractère ionique sous-jacent.

17. Composition médicamenteuse pour le traitement de la peau, des muqueuses ou du tube digestif, caractérisée en ce qu'elle contient un produit antimicrobien conforme à l'une des revendications 14 à 16.

18. Composition pour la décontamination microbienne de produits ou matériaux, caractérisée en ce qu'elle contient un produit antimicrobien conforme à l'une des revendications 14 à 16.

19. Dentifrice, caractérisé en ce qu'il contient un produit antimicrobien conforme à l'une des revendications 14 à 16.

## Patentansprüche

1. Verfahren zur Herstellung eines teilchenförmigen antimikrobiellen Produkts ausgehend von dem System antimikrobielles Enzym Lactoperoxydase/ Sauerstoffspender/gegebenenfalls oxidationsfähiges Substrat, dem sogenannten System LP, bei dem der Sauerstoffspender entweder aus Peroxid oder aus reaktiven Stoffen, von denen einer ein Enzym sein kann, oder aus Mikroorganismen besteht, wobei das besagte Verfahren die Aufgabe hat, die physikalisch-chemischen Eigenschaften des Systems LP abzuändern, um es in der Form von in wäßrigem Medium unlöslichen Teilchen zu präsentieren, und zwar ist das besagte Verfahren dadurch gekennzeichnet,
. daß man mindestens das Enzym Lactoperoxydase des Systems LP an einem teilchenförmigen Träger verankert, bei dem jedes Teilchen einen aus einem hydrophilen Polysaccharid-Polymer bestehenden Kern umfaßt, einer langkettige Fettsäuren umfassenden ersten lipiden Schicht, die durch Hydroxyfunktionen bzw. derivierte Funktionen chemisch mit dem Kern verbunden ist, und einer zweiten phospholipiden Schicht besteht, so daß die Enzymmoleküle in der zweiten phospholipiden Schicht und/oder in der ersten lipiden Schicht eingefügt sind,
. daß man den besagten teilchenförmigen Träger und die in dem besagten teilchenförmigen Träger nicht integrierten Moleküle des Systems LP in nichtwäßrigem Medium einbettet.

2. Herstellungsverfahren nach Anspruch 1, bei dem der Sauerstoffspender ein Enzym Oxydase als Wirkstoff enthält, dadurch gekennzeichnet, daß man an dem teilchenförmigen Träger auch die besagten Oxydase-Enzymmoleküle verankert.

3. Verfahren nach einem der Ansprüche 1 oder 2 zur Herstellung eines durch Lactoferrin LF verstärkten Systems LP, dadurch gekennzeichnet, daß man das LF an dem teilchenförmigen Träger verankert.

4. Verfahren nach einem der Ansprüche 1, 2 oder 3 für die Herstellung eines Systems LP, das als Mediumsschutz ein Enzym Superoxydismutase SOD enthält, dadurch gekennzeichnet, daß man das SOD an dem teilchenförmigen Träger verankert.

5. Herstellungsverfahren nach einem der Ansprüche 1, 2, 3 oder 4, dadurch gekennzeichnet, daß man auf die Oberfläche des Polysaccharidkerns gleichzeitig mit der Bildung der ersten lipiden Schicht ionische Funktionen aufpfropft, um dieser lipiden Schicht eine ionische Beschaffenheit zu vermitteln.

6. Herstellungsverfahren nach einem der Ansprüche 1, 2, 3, 4 oder 5, dadurch gekennzeichnet, daß man elektrisch geladene amphiphile Moleküle in die zweite phospholipide Schicht des teilchenförmigen Trägers einfügt, um den Teilchen eine elektrische Ladung zu vermitteln.

7. Herstellungsverfahren nach einem der Ansprüche 1 bis 6, bei dem die Verankerung des bzw. der zu verankernden Bestandteile (Enzym(e) und gegebenenfalls LF, SOD) an dem betreffenden Träger dadurch bewirkt wird,
. daß man zwecks Bildung eines acylierten Kerns in einer ersten Stufe in einem Polysaccharide nicht lösenden aprotischen Medium die lipide Schicht um den Polysaccharidkern herum aufpfropft,
. daß man danach einerseits die Phospholipide der zweiten Schicht und andererseits das bzw. die zu verankernden Bestandteile mit dem besagten acylierten Kern in Kontakt bringt und die Gesamtheit bei einer der Umwandlungstemperatur der Phospholipide nahen Temperatur hydratisiert.

8. Herstellungsverfahren nach den Ansprüchen 5 und 7 gemeinsam, dadurch gekennzeichnet, daß das Aufpfropfen der lipiden Schicht und ihrer ionischen Funktionen auf den Saccharidkern darin besteht, daß man den Polysaccharidkern mit einem Gemisch aus einem Fettsäurechlorid und einem Carboxyldiacidanhydrid, insbesondere einem einen geringeren Anteil bildenden Bernstoffsäureanhydrid, zur Reaktion bringt.

9. Herstellungsverfahren nach einem der Ansprüche 1 bis 8, dadurch gekennzeichnet, daß man die Teilchen des teilchenförmigen Trägers nach der Verankerung mit einer Oberflächenschicht von Polyolosid oder kolloidalem Stoff umgibt.

10. Herstellungsverfahren nach einem der Ansprüche 1 bis 9, bei dem der Kern von einem Polysaccharidpolymer der folgenden Gruppe - Amidon, Dextran, Cellulose - ausgehend erzeugt wird.

11. Herstellungsverfahren nach einem der Ansprüche 1 bis 10, bei dem die erste lipide Schicht durch Reaktion eines durch ein tertiäres Amin katalysierten Säurechlorids bzw. -anhydrids erzeugt wird, wobei die besagten Bestandteile gleichzeitig mit den Saccharidkernen in dem Dichlormethan dispergiert werden.

12. Herstellungsverfahren nach Anspruch 11, dadurch gekennzeichnet, daß man zur Erzeugung der ersten Schicht ein Chlorid oder Anhydrid einer langkettigen Fettsäure mit zwischen 14 und 22 und insbesondere mit 18 Gliedern benutzt.

13. Herstellungsverfahren nach einem der Ansprüche 1 bis 12, bei dem die zweite phospholipide Schicht von Phospholipiden tierischen oder pflanzlichen Ursprungs wie Soyalecithin, Eierlecithin oder Milchlecithin ausgehend erzeugt wird.

14. Teilchenförmiges antimikrobielles Produkt, das ein Enzym Lactoperoxidase, einen möglicherweise ein Enzym als Wirkstoff enthaltenden Sauerstoffspender und gegebenenfalls ein oxidationsfähiges Substrat umfaßt, dadurch gekennzeichnet, daß mindestens das Enzym Lactoperoxidase des Systems an einem teilchenförmigen Träger verankert ist, bei dem jedes Teilchen einen aus einem hydrophilen Polysaccharidpolymer bestehenden Kern, eine chemisch mit dem Kern durch Hydroxyfunktionen oder derivierte Funktionen verbundene erste lipide Schicht und eine zweite phospholipide Schicht umfaßt, wobei die besagten Enzymmoleküle in der zweiten phospholipiden Schicht und/oder in der ersten lipiden Schicht eingefügt sind.

15. Teilchenförmiges antimikrobielles Produkt nach Anspruch 14, dadurch gekennzeichnet, daß es an dem teilchenförmigen Träger verankerte Moleküle wenigstens einer der folgenden Verbindungen - Lactoferrin LF, Superoxydismutase SOD - umfaßt.

16. Teilchenförmiges antimikrobielles Produkt nach einem der Ansprüche 14 oder 15, dadurch gekennzeichnet, daß die erste lipide Schicht eine zugrundeliegende ionische Beschaffenheit aufweist.

17. Heilmittelverbindung zur Behandlung der Haut, der Schleimhäute oder des Verdauungskanals, dadurch gekennzeichnet, daß sie ein antimikrobielles Produkt gemäß der Ansprüche 14 bis 16 enthält.

18. Verbindung für mikrobielle Dekontaminierung von Produkten bzw. Materialien, dadurch gekennzeichnet, daß sie ein den Ansprüche 14 bis 16 gemäßes antimikrobielles Produkt enthält.

19. Zahnreinigungsmittel, dadurch gekennzeichnet, daß es ein einem der Ansprüche 14 bis 16 gemäßes antimikrobielles Produkt enthält.

## Claims

1. Method of preparation of a particulate antimicrobial product from the antimicrobial system of the lactoperoxydase enzyme/oxygen donor/oxidizable substrate (if needed), called the LP system, in which the oxygen donor comprises either a peroxide, a reactive substance of which one may be an enzyme, or micro-organisms, said process being intended to modify the physicochemical properties of the LP system for arranging it in the form of insoluble particles in an aqueous medium, the process comprising:
bonding at least the enzyme lactoperoxidase of the LP system to a particulate vector each particle of which comprises a nucleus formed of a hydrophilic polysaccharidic polymer, a first lipidic layer comprising long chain fatty acids, chemically bound to the nucleus by hydroxy functional groups or derivatives thereof, and a second phospholipidic layer, of a kind such that the molecules of enzyme are inserted into the second phospholipidic layer and/or into the first lipidic layer,
conditioning the aforesaid particulate vector and the molecules of the LP system not integrated in said particulate vector in a non-aqueous medium.

2. Preparation process according to Claim 1, in which the oxygen donor contains as an active substance an oxidase enzyme, characterized in additionally bonding onto the particulate vector said oxidase enzyme molecules.

3. Process according to Claim 1 for the preparation of an LP system strengthened by lactoferrine LF, characterized in bonding the LF onto the particulate vector.

4. Process according to Claim 1 for the preparation of an LP system containing as a protector of the medium an enzyme superoxidase SOD, characterized in bonding the SOD onto the particulate vector.

5. Process for preparation according to Claim 1, characterized in grafting onto the surface of the polysaccharide nucleus ionic functional groups at the same time as the formation of the first lipidic layer for confering an ionic character upon said lipidic layer.

6. Preparation process according to Claim 1 characterized in inserting electrically charged amphophilic molecules into the second phospholipidic layer on the particulate vector, in order to confer an electric charge on the particles.

7. Preparation process according to Claim 5 in which the bonding to the particulate vector of the one or more bonding elements (enzyme(s) and if desired LF, SOD) is produced: initially by grafting the lipidic layer around the polysaccharide nucleus in an aprotic medium which is a non-solvent for the polysaccharides for forming an acylic nucleus, then placing in the presence of said acylated nucleus, the phospholipids of the second layer, the one or more bonding elements, and hydrating the composite at a temperature in the vicinity of the transition temperature of the phospholipids.

8. Preparation process according to Claim 7, characterized in grafting on the saccharidic nucleus of the lipidic layer and its ionic functions comprises causing a reaction of the polysaccharidic nucleus with a mixture of a fatty acid chloride and a dicarboxylic acid anhydride, in particular succinic anhydride in a minor amount.

9. Preparation process according to Claim 1, characterized in, after the bonding, covering the particles of the particulate vector by means of a surface layer of polyoloside or of a colloidal substance.

10. Preparation process according to Claim 1 in which the nucleus is produced from a polysaccharidic polymer selected from the following group: starch, dextran, cellulose.

11. Preparation process according to Claim 1 in which the first lipidic layer is produced by the reaction of a chloride or an acid anhydride, catalyhzed by a tertiary amine, these compounds being dispersed in dichloromethane at the same time as the saccharidic nuclei.

12. Preparation process according to Claim 11, characterized in using for producing the first layer a chloride or an anhydride of a long chain fatty acid betweena 14 and 22 andparticularly equal to 18.

13. Preparation process according to Claim 1 in which the second phospholipidic layer is produced from phospholipids of animal or vegetable origin such as soy lecithin, egg lecithin, or milk lecithin.

14. Particulate antimicrobial product comprising a lactoperoxidase enzyme, an oxygen donor containing an enzyme as a reactive substance, and if desired an oxidizable substrate, characterized in that at least the lactoperoxidase enzyme of the system is bonded to a particulate vector of which each particle comprises a nucleus constituted by a hydrophilic polysaccharidic polymer, a first lipidic layer clhemically bonded to the nucleus by hydroxy functional groups or derivatives thereof, and a second phospholipidic layer, said enzyme molecules being inserted into the second phospholipidic layer and/or in the first lipidic layer.

15. Particulate antimicrobial product according to Claim 14, characterized in that it comprises, bonded to the particulate vector, molecules of at least one of the following compounds: lactoferrine LF, superoxydismutase SOD.

16. Particulate antimicrobial product according to Claim 14 characterized in that the first lipidic layer has a subjacent ionic character.

17. Medicamentous composition for the treatment of skin, mucous membranes or of the digestive tract, characterized in that it contains an antimicrobial product as in Claim 14.

18. Composition for the microbial decontamination of products or materials, characterized in that it contains an antimicrobial product conforming to Claim 14.

19. Dentifrice, characterized in that it contains an antimicrobial product according to Claim 14.
